(19) Europäisches Patentamt
European Patent Office
Office européen des brevets

(11) Numéro de publication : **0 036 352**
**B1**

(12) **FASCICULE DE BREVET EUROPÉEN**

(45) Date de publication du fascicule du brevet : **01.06.83**

(21) Numéro de dépôt : **81400328.1**

(22) Date de dépôt : **04.03.81**

(51) Int. Cl.³ : **C 07 C 17/20**, C 07 C 21/24, C 07 C 25/13, C 07 C 63/10, C 07 C 51/363, B 01 J 10/00, B 01 J 19/24

(54) **Procédé de préparation de trifluorométhylbenzènes à partir des trichloro- ou tribromo-méthylbenzènes correspondants.**

(30) Priorité : **17.03.80 FR 8005877**

(43) Date de publication de la demande : **23.09.81 Bulletin 81/38**

(45) Mention de la délivrance du brevet : **01.06.83 Bulletin 83/22**

(84) Etats contractants désignés : **AT BE CH DE FR GB IT LI LU NL SE**

(56) Documents cités :
**DE A 1 618 390**
**FR A 2 137 744**
**FR A 2 295 004**
**US A 2 181 554**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Ramanadin**
**Montée de la Daude**
**F-30100 Alès (FR)**
Inventeur : **Seigneurin, Laurent**
**3, avenue du Parc**
**F-30340 Salindres (FR)**

(74) Mandataire : **Cazes, Jean-Marie et al**
**RHONE-POULENC RECHERCHES Service Brevets Chimie et Polymères 25, quai Paul Doumer**
**F-92408 Courbevoie Cedex (FR)**

**EP 0 036 352 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé de préparation de trifluorométhylbenzènes à partir des trichloro- ou tribromo-méthylbenzènes correspondants

La présente invention concerne un procédé pour la préparation de trifluorométhylbenzènes à partir des trichloro- ou tribromo-méthylbenzènes correspondants. Elle concerne plus particulièrement la réaction en continu des trichloro- ou tribromo-méthylbenzènes avec de l'acide fluorhydrique.

Au sens de la présente invention, on entend par trifluorométhylbenzènes, les composés benzéniques comportant au moins un substituant trifluorométhyle et, éventuellement, un autre substituant.

On connaît dans l'art antérieur le brevet des Etats-Unis d'Amérique n° 3 966 832 qui décrit la préparation de dérivés du benzène contenant au moins un groupement trifluorométhyle. Selon ce procédé, on introduit en continu dans un autoclave sous une pression supérieure à 20 atmosphères, le trichlorométhylbenzène et l'acide fluorhydrique, ce dernier étant en excès d'au moins 25 moles % par rapport à la stœchiométrie ; après que le mélange réactionnel a atteint une hauteur déterminée, ou bien on retire le mélange liquide obtenu par une soupape à un débit correspondant au débit d'alimentation, ou bien on fait passer le mélange dans un ou plusieurs autres autoclaves et on le retire du dernier autoclave de la cascade constituant l'installation. Le ou les autoclaves sont maintenus à une température comprise entre 80 °C et 150 °C. On initie la réaction en préchargeant l'autoclave avec de l'acide fluorhydrique seul ou en mélange homogène avec le trichlorométhylbenzène et de l'acide chlorhydrique à une pression d'au moins 20 atmosphères absolues ceci dans le but d'éviter le danger d'explosion lors du démarrage de la réaction qui pourrait être provoquée par une augmentation de pression trop rapide. Pour obtenir un mélange suffisamment homogène nécessaire à la bonne marche en continu du procédé, il est généralement nécessaire d'opérer sous une pression de 30 à 50 atmosphères. D'après les propres constatations de la demanderesse, cette pression sera d'autant plus élevée que le noyau benzénique du trichlorométhylbenzène sera plus substitué.

Ceci est important dans la mesure où des produits intéressants au plan industriel comme intermédiaires pour la préparation d'herbicides sont justement des trifluorométhylbenzènes comportant un ou plusieurs substituants chlorés sur le noyau benzénique.

On connaît encore dans l'art antérieur la demande de brevet allemand 1 618 390 qui décrit la mise en réaction dans un réacteur tubulaire d'un dérivé benzénique trichlorométhylé avec un excès d'HF d'au moins 1/3 par rapport à la stœchiométrie à une température de 90-130 °C et sous une pression de 30-50 atmosphères.

Le gros excès d'acide fluorhydrique utilisé dans l'art antérieur, implique que le mélange réactionnel contient une quantité importante d'acide fluorhydrique qu'il est nécessaire de séparer et de recycler à l'alimentation du réacteur. Cette quantité d'acide fluorhydrique est nécessaire si l'on veut obtenir une transformation quasi quantitative. Il est nécessaire dans cette même optique d'utiliser dans certains cas une cascade d'autoclave comme cela est décrit dans le brevet précité.

Il apparaît clairement que les inconvénients de l'art antérieur dans le cadre d'un procédé de préparation en continu de trifluorométhylbenzènes à partir des trichlorométhylbenzènes correspondants peuvent principalement s'analyser en trois points : il est tout d'abord nécessaire, selon l'art antérieur, d'opérer avec un gros excès d'acide fluorhydrique. Pour ne pas détruire l'économie du procédé, il est nécessaire de séparer et de recycler cette quantité excédentaire ce qui implique un appareillage important au niveau d'une installation industrielle.

En second lieu, pour assurer une bonne homogénéisation, il est nécessaire d'opérer sous une pression très élevée, ce qui, là aussi, conduit à un appareillage sophistiqué et important.

Enfin, ce problème d'appareillage est particulièrement crucial quand il devient nécessaire selon la matière première, d'utiliser une pluralité d'autoclaves en série sous pression.

La présente invention pallie les inconvénients de l'art antérieur.

La présente invention a pour objet un procédé en continu de préparation d'un trifluorométhylbenzène à partir du trichloro- ou tribromo-méthylbenzène correspondant par action sur ce dernier d'acide fluorhydrique, caractérisé en ce que l'on met en œuvre la réaction, en faisant passer à contre-courant un courant ascendant d'acide fluorhydrique en phase gazeuse à travers une pluralité de couches liquides successives mobiles du trichloro- ou tribromo-méthylbenzène, la pression étant comprise entre $5 \cdot 10^5$ et $20 \cdot 10^5$ Pa (5 et 20 atmosphères) et le rapport molaire de l'acide fluorhydrique au trichloro ou tribromométhylbenzène étant compris entre 3 et 4.

Selon un mode de réalisation préférentiel de l'invention, la pression est comprise entre $8 \cdot 10^5$ et $15 \cdot 10^5$ Pa (8 et 15 atmosphères).

Selon un autre mode de réalisation préférentiel de l'invention, on met en œuvre la réaction en utilisant l'acide fluorhydrique en quantité telle que le rapport molaire de l'acide fluorhydrique ou trichloro- ou tribromo-méthylbenzène est compris entre environ 3 et 3,5.

La température de réaction est de préférence comprise entre 80 °C et 150 °C et encore plus préférentiellement entre 90 et 120 °C.

Le nombre de couches liquides mobiles est choisi, de préférence, de telle sorte que le courant gazeux après avoir traversé la dernière couche liquide contienne principalement de l'acide chlorhydrique pratiquement exempt d'acide fluorhydrique et que le liquide issu de la première couche liquide traversée par l'acide fluorhydrique gazeux contienne principalement le dérivé trifluorométhylé pratiquement

exempt de dérivé trichloro- ou tribromo-méthylé. Ce double objectif peut être plus étroitement contrôlé en modifiant dans le coùrs du procédé de rapport molaire de l'acide fluorhydrique au trichloro- ou tribromo-méthylbenzène, tout en restant à l'intérieur des limites précitées.

Il apparaît clairement que l'invention permet donc, tout en obtenant un rendement en trifluorométhylbenzène par rapport au trichloro- ou tribromo-méthylbenzène correspondant supérieur à 95 %, d'opérer à des pressions très notablement plus basses que celles qu'il était nécessaire de maintenir dans les procédés de l'art antérieur et d'utiliser des quantités d'acide fluorhydrique considérablement plus faibles et généralement voisines de la stœchiométrie. Le procédé selon l'invention permet d'autre part d'obtenir séparément le produit trifluorométhylé recherché et l'acide chlorhydrique sous-produit ce qui simplifie les traitements ultérieurs.

Le débit des réactifs est de préférence réglé de telle façon que le temps de contact total du gaz acide fluorhydrique et du liquide soit compris entre environ 10 et 100 mn. Encore plus préférentiellement, il est compris entre environ 15 et environ 75 mn. Ce temps de contact est défini comme le rapport du volume de liquide des diverses couches liquides au débit d'alimentation du trichloro- ou dibromo-méthylbenzène mis en œuvre.

L'invention est plus particulièrement destinée à l'obtention des trifluorométhylbenzènes de formule générale :

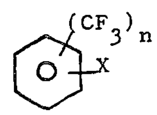

à partir des trichloro- ou tribromo-méthylbenzènes correspondants, de formules :

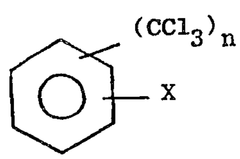
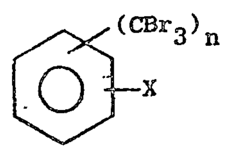

où X représente au moins un substituant inerte dans les conditions de la réaction, comme par exemple : H, Cl, F, Br, COF, $CF_3$, et où n est supérieur ou égal à 1, et de préférence, égal à 1 ou 2. X peut également représenter tout substituant transformable, selon un processus connu dans les conditions de la réaction, comme COCl ou COBr qui se transforme comme cela est connu, en COF.

On peut citer comme exemples de trifluorométhylbenzènes pouvant être préparés par le procédé selon l'invention, le trifluorométhylbenzène, le chloro-3 trifluorométhylbenzène, le chloro-4 trifluorométhylbenzène, le dichloro-3,4 trifluorométhylbenzène, le fluoro-3 trifluorométhylbenzène, le parabromotrifluorométhylbenzène, le parafluorotrifluoro-méthylbenzène, le métabis-trifluorométhylbenzène, le parabis-trifluorométhylbenzène, le fluorure de méta-trifluorométhylbenzoyle, le fluorure d'orthotrifluorométhyl benzoyle.

L'invention va être maintenant plus complètement décrite en référence à la figure jointe qui représente un dispositif qui peut être utilisé pour la mise en œuvre du procédé selon l'invention.

Ce dispositif comprend une colonne réactionnelle A, une colonne de dévésiculage B, un bouilleur d'acide fluorhydrique C et un décanteur D.

La colonne réactionnelle A contient fixés sur un axe 1 une pluralité de plateaux 2 comportant plusieurs ouvertures de faible diamètre 3. Chaque plateau est traversé par une tubulure 4. La colonne A, le bouilleur C et le décanteur D sont munis de doubles enveloppes 5, 6 et 7 dans lesquelles circule un fluide caloporteur.

On introduit en continu en 8 le dérivé trichloro- ou tribromométhylé liquide. Le liquide forme d'abord une couche liquide sur le plateau 2 supérieur. Lorsque le niveau du liquide arrive à hauteur de l'extrémité supérieure de la canalisation 4, la première couche 9 est formée et le liquide emprunte cette canalisation et se déverse sur le deuxième plateau 2 où il forme de la même façon une deuxième couche liquide 9 et ainsi de suite jusqu'au plateau inférieur.

Simultanément à l'introduction du dérivé trichloro- ou tribromo-méthylé on introduit en continu en 10 de l'acide fluorhydrique liquide qui pénètre dans le bouilleur C où il se vaporise, la température y étant maintenue entre environ 80 °C et environ 150 °C. Le gaz acide fluorhydrique ainsi formé pénètre dans la colonne A et traverse les couches liquides 9 en passant par les ouvertures 3 des plateaux 2. Ces ouvertures sont calculées pour que le liquide soit maintenu au-dessus du plateau par la simple pression du gaz.

Après avoir traversé toutes les couches liquides, le gaz qui est constitué essentiellement d'acide chlorhydrique pénètre dans la colonne de dévésiculage où il est débarrassé du liquide organique entraîné.

Lorsque la pression atteint la valeur retenue on détend le gaz chlorhydrique par la vanne 11. Le gaz chlorhydrique qui contient une très faible proportion d'acide fluorhydrique est envoyé, en continu, par 12 dans une étape classique de rectification.

Le liquide issu de la dernière couche 9 et qui est constitué essentiellement par le dérivé trifluorométhylé, est recueilli dans le décanteur D où la petite quantité d'acide fluorhydrique entraînée décante et forme une couche supérieure 14 qui sera vaporisée en même temps que le HF frais qui est introduit en 10.

On soutire en continu, en 13, le produit de la réaction qui contient une très faible proportion de dérivé trichloro- ou tribromo-méthylé et on l'envoie vers une étape classique de distillation.

Comme cela a été précisé précédemment, le nombre de couches liquides, leur volume et les débits des réactifs sont réglés de telle façon à minimiser la présence d'acide fluorhydrique dans les gaz de sortie en 12 et la présence de dérivé trichloro- ou tribromo-méthylé dans le liquide en 13.

D'autres avantages et caractéristiques de l'invention apparaîtront plus clairement à la lecture des exemples qui vont suivre.

Ces exemples ne sauraient être considérés comme limitant de façon quelconque l'invention.

## Exemple 1

Préparation de trifluorométhylbenzène $C_6H_5\text{-}CF_3$ à partir de trichlorométhylbenzène $C_6H_5\text{-}CCl_3$

On opère dans un appareillage tel que représenté à la figure unique dont la colonne A est une colonne de 3 mètres de haut, de 6,5 cm de diamètre et comportant 15 plateaux ayant chacun 31 trous de 2 mm de diamètre répartis uniformément selon un pas carré. On introduit 870 g/h (43,5 moles/h) d'HF liquide et 2 820 g/h de trichlorométhylbenzène technique, contenant 2 737 g de trichlorométhylbenzène pur (14 moles/h). Le HF se vaporise dans le bouilleur C où l'on maintient une température de 90-92 °C et traverse les 15 couches liquides de 6 cm de hauteur.

On maintient dans la colonne A une pression de 9 atmosphères et une température de 90-92 °C.

On soutire 2 110 g/h d'un mélange organique liquide dont l'analyse révèle la composition pondérale suivante :

— $C_6H_5CF_3$ : 95,5 %
— $C_6H_5CF_2Cl$ : 1,3 %
— $C_6H_5CFCl_2$ : 0,25 %
— $C_6H_5CCl_3$ : 0,002 %

le reste étant constitué par les impuretés de la matière première.

Cela correspond à un rendement molaire en $C_6H_5CF_3$ par rapport au $C_6H_5CCl_3$ de 95,7 % et à un taux de fluoration

$$\frac{\text{nombre d'atomes de fluor fixés sur le groupement } CCl_3}{\text{nombre d'atomes de chlore substituables}} \times 100$$

de 99,45 %.

On recueille en 12 un gaz contenant 3,9 % en moles d'HF et 96,08 % en moles d'HCl.

## Exemple 2

Préparation du parachlorotrifluorométhylbenzène $p\text{-}ClC_6H_4CF_3$ à partir du parachlorotrichlorométhylbenzène $p\text{-}ClC_6H_4CCl_3$

Dans le même appareillage que celui décrit à l'exemple 1, on introduit :

— 3 220 g/h (14 moles) de $p\text{-}ClC_6H_4CCl_3$ pur
— 980 g/h (49 moles) de HF liquide.

La température dans l'ensemble de l'appareillage est maintenue à 110 °C, la pression étant de 14,5 atmosphères.

On recueille 2 530 g/h d'un liquide organique contenant, en poids :

— 98,5 % de $p\text{-}ClC_6H_4CF_3$
— 1,4 % de $p\text{-}ClC_6H_4CF_2Cl$
— 0,07 % de $p\text{-}ClC_6H_4CFCl_2$
— des traces de $p\text{-}ClC_6H_4CCl_3$

le reste étant constitué par les impuretés de la matière de départ.

Le rendement molaire en p-$ClC_6H_4CF_3$ par rapport au p-$ClC_6H_4CCl_3$ est de 98,6 %. Le taux de fluoration tel que défini à l'exemple 1 est de 99,5 %. On recueille en 12 un gaz contenant en moles 14,7 % d'HF et 85,3 % d'HCl.

Exemple 3

Préparation de dichloro-3,4 trifluorométhylbenzène à partir de dichloro-3,4 trichlorométhylbenzène

Dans le même appareillage que dans l'exemple 1, on introduit 3 703 g/h (14 moles) de dichloro-3,4 trichlorométhylbenzène préalablement purifié et 900 g/h (4,5 moles) d'HF liquide.

La température de l'ensemble est maintenue à 120 °C sous une pression de 15 atmosphères.

On recueille 2 385,5 g/h d'un mélange dont l'analyse révèle la composition pondérale suivante :

— 97,5 % de 3,4-$Cl_2C_6H_3CF_3$
— 1,62 % de 3,4-$Cl_2C_6H_3CF_2Cl$
— 0,8 % de 3,4-$Cl_2C_6H_3CFCl_2$
— 0,08 % de 3,4-$Cl_2C_6H_3CCl_3$

Le rendement molaire en 3,4 $Cl_2C_6H_3CF_3$ par rapport au 3,4 $Cl_2C_6H_3CCl_3$ est de 98,34 %. Le taux de fluoration est de 99,36 %. On recueille en 12 un gaz contenant en moles 92,74 % d'HCl et 7,25 % d'HF.

Exemple 4

Préparation du fluorure d'orthotrifluorométhylbenzoyle $CF_3C_6H_4COF$ à partir de chlorure d'orthotrichlorométhylbenzoyle $CCl_3C_6H_4COCl$.

On introduit dans le même appareillage que dans l'exemple 1, 3 612 g/h (14 moles) de $CCl_3C_6H_4$-COCl liquide et 1 240 g/h (62 moles) de HF liquide.

La température de l'ensemble est maintenue à 100 °C sous une pression de 15 atmosphères.

On recueille 2 695 g/h d'un mélange organique liquide dont la composition pondérale est la suivante :

— 96,9 % de $CF_3C_6H_4COF$
— 3,0 % de $CF_2ClC_6H_4COF$.

et des traces de $CFCl_2C_6H_4COF$ et de $CCl_3C_6H_4COF$.

Le rendement molaire en $CF_3C_6H_4COF$ par rapport au $CCl_3C_6H_4COCl$ est de 97,14 %. Le taux de fluoration est de 99 %.

On recueille en 12, un gaz contenant en moles 89,6 % d'HCl et 10,3 % d'HC.

**Revendications**

1. Procédé continu de préparation d'un trifluorométhylbenzène à partir du trichloro- ou tribromométhylbenzène correspondant par action sur ce dernier d'acide fluorhydrique caractérisé en ce que l'on met en œuvre la réaction en faisant passer à contre-courant un courant ascendant d'acide fluorhydrique en phase gazeuse à travers une pluralité de couches liquides successives mobiles du trichloro- ou tribromo-méthylbenzène, la pression étant comprise entre $5 \cdot 10^5$ et $20 \cdot 10^5$ Pa (5 et 20 atmosphères) et le rapport molaire de l'acide fluorhydrique au trichloro- ou tribromométhylbenzène étant compris entre 3 et 4.

2. Procédé selon la revendication 1, caractérisé en ce que l'on met en œuvre la réaction à une température comprise entre 80 °C et 150 °C.

3. Procédé selon la revendication 2, caractérisé en ce que la température est comprise entre 90 °C et 120 °C.

4. Procédé selon la revendication 1, caractérisé en ce que la pression est comprise entre $8 \cdot 10^5$ et $15 \cdot 10^5$ Pa (8 et 15 atmosphères).

5. Procédé selon la revendication 1, caractérisé en ce que le rapport molaire est compris entre 3 et 3,5.

6. Procédé selon la revendication 1, caractérisé en ce que le rapport du volume total de couches liquides au débit d'alimentation du trichloro- ou tribromo-méthylbenzène est compris entre 10 et 100.

7. Procédé selon la revendication 6, caractérisé en ce que le rapport est compris entre 15 et 75.

**Claims**

1. Continuous process for the preparation of a trifluoromethylbenzene from the corresponding

trichloromethylbenzene or tribromomethylbenzene by reaction of hydrofluoric acid with the latter, characterised in that the reaction is carried out by passing a rising stream of hydrofluoric acid in the gas phase, in counter-current, through a plurality of successive moving liquid layers of the trichloromethylbenzene or tribromomethylbenzene, the pressure being between $5 \cdot 10^5$ and $20 \cdot 10^5$ Pa (between 5 and 20 atmospheres) and the molar ratio of the hydrofluoric acid to the trichloromethylbenzene or tribromomethylbenzene being between 3 and 4.

2. Process according to Claim 1, characterised in that the reaction is carried out at a temperature of between 80 °C and 150 °C.

3. Process according to Claim 2, characterised in that the temperature is between 90 °C and 120 °C.

4. Process according to Claim 1, characterised in that the pressure is between $8 \cdot 10^5$ and $15 \cdot 10^5$ Pa (between 8 and 15 atmospheres).

5. Process according to Claim 1, characterised in that the molar ratio is between 3 and 3.5.

6. Process according to Claim 1, characterised in that the ratio of the total volume of liquid layers to the feed rate of the trichloromethylbenzene or tribromomethylbenzene is between 10 and 100.

7. Process according to Claim 6, characterised in that the ratio is between 15 and 75.

**Ansprüche**

1. Kontinuierliches Verfahren zur Herstellung eines Trifluormethylbenzols aus dem entsprechenden Trichlor- oder Tribom-methylbenzol durch Einwirkung von Fluorwasserstoffsäure auf letzteres, dadurch gekennzeichnet, daß man zur Durchführung der Reaktion einen aufsteigenden Strom von Fluorwasserstoffsäure in der Gasphase im Gegenstrom durch mehrere aufeinanderfolgende, in Bewegung befindliche Flüssigkeitsschichten von Trichlor- oder Tribrom-methylbenzol leitet, wobei der Druck $5 \cdot 10^5$ bis $20 \cdot 10^5$ Pa (5 bis 20 At.) und das Molverhältnis von Fluorwasserstoff zum Trichlor- oder Tribrom-methylbenzol 3 bis 4 beträgt.

2. Verfahren nach Anspruch 1, dadurch gekennzeichnet, daß man die Reaktion bei einer Temperatur zwischen 80 und 150 °C durchführt.

3. Verfahren nach Anspruch 2, dadurch gekennzeichnet, daß die Temperatur 90 bis 120 °C beträgt.

4. Verfahren nach Anspruch 1, dadurch gekennzeichnet, daß der Druck $8 \cdot 10^5$ bis $15 \cdot 10^5$ Pa (8 bis 15 At.) beträgt.

5. Verfahren nach Anspruch 1, dadurch gekennzeichnet, daß das Molverhältnis zwischen 3 und 3,5 liegt.

6. Verfahren nach Anspruch 1, dadurch gekennzeichnet, daß das Verhältnis des Gesamtvolumens der Flüssigkeitsschichten zum zugeführten Trichlor- oder Tribrom-methylbenzol zwischen 10 und 100 liegt.

7. Verfahren nach Anspruch 6, dadurch gekennzeichnet, daß das Verhältnis zwischen 15 und 75 liegt.

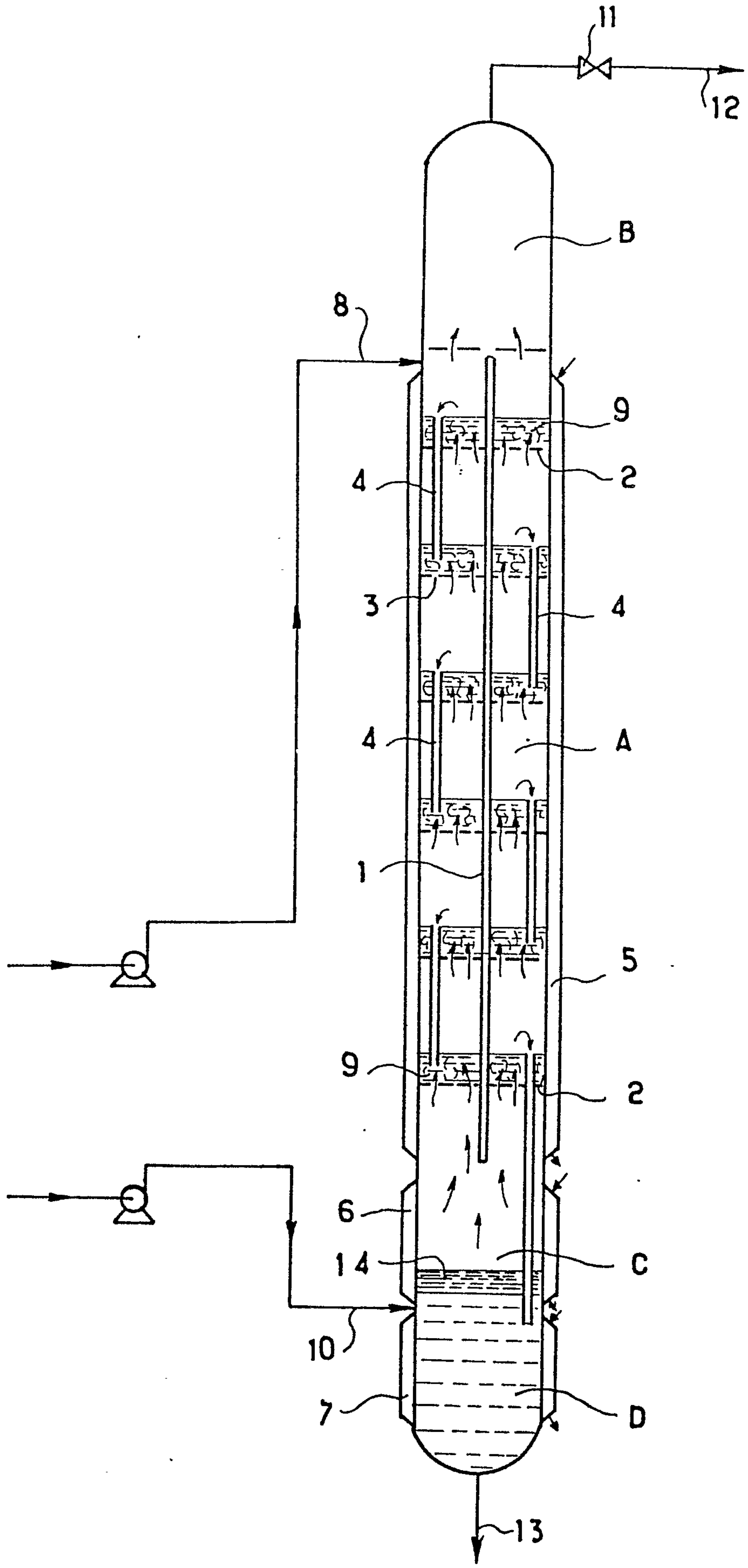
11
12
B
8
9
2
4
3
4
4
A
1
5
9
2
6
14
C
10
7
D
13